# EUROPÄISCHE PATENTSCHRIFT

(11) **EP 1 768 764 B1**
(45) Veröffentlichungstag und Bekanntmachung des Hinweises auf die Patenterteilung: **29.08.2012**
(21) Anmeldenummer: 05766105.0
(22) Anmeldetag: 07.07.2005
(51) Int. Cl.: B01D 53/14, C07C 319/20

(54) **VERFAHREN ZUR REINIGUNG VON CO2-GASSTRÖMEN**
METHOD FOR PURIFYING CO2 GASFLOW
PROCEDE D'EPURATION DE COURANTS GAZEUX DE CO2

(30) Priorität: 22.07.2004 DE 102004035465
(43) Veröffentlichungstag der Anmeldung: 04.04.2007
(73) Patentinhaber: Evonik Degussa GmbH, 45128 Essen (DE)
(72) Erfinder: HASSELBACH, Hans-Joachim, 63571 Gelnhausen (DE); VANROBAEYS, Jose, B-2920 Kalmthout (BE); KÖRFER, Martin, B-2920 Kalmthout-Heide (BE)
(86) Internationale Anmeldenummer: PCT/EP2005/007323
(87) Internationale Veröffentlichungsnummer: WO 2006/010448

(56) Entgegenhaltungen:
- EP-A- 0 780 370
- US-A- 4 319 044

## Beschreibung

Die Erfindung betrifft ein Verfahren zur Reinigung von CO₂-Abgasströmen von chemischen Verbindungen und die Rückführung der gereinigten Gasströme in den Produktionsprozess.

Derartige Abgase treten insbesondere bei der Herstellung von Methionin auf und enthalten neben Methylmercaptan im allgemeinen auch Ammoniak, Blausäure und Wasserdampf.

Ein solcher Methionin-Prozess wird zum Beispiel in der EP-B 0780370 = US 5,770,769 und US 5990349 beschrieben.

Die zugrundeliegenden Prozessschritte lassen sich wie folgt veranschaulichen:
Schritt 1: Bildung von 5-(2-Methylmercapto)hydantoin 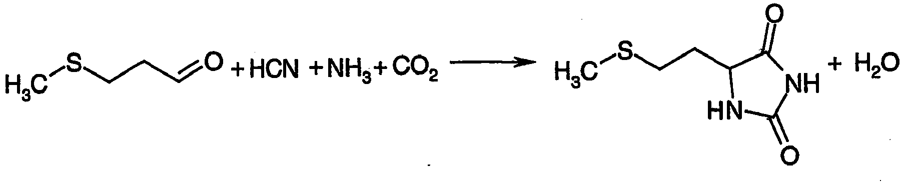
Schritt 2: Hydrolyse des Hydantoins unter Bildung des Methionin-Kalium-Salzes 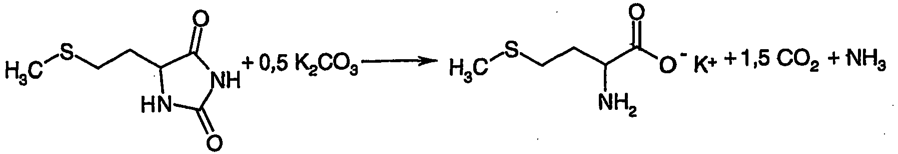
Schritt 3: Strippung von NH₃ und CO₂ aus der Hydrolysemischung. 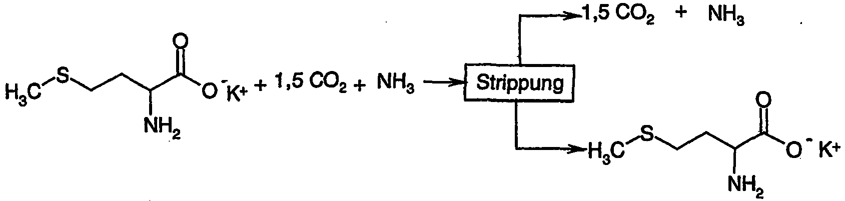
Schritt 4: Freisetzung von festem Methionin aus seinem Kalium-Salz 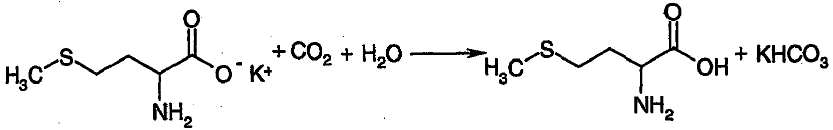
Schritt 5: Trennung von festem Methionin und Mutterlauge 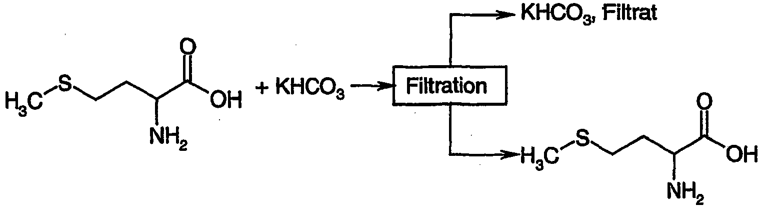
Schritt 6: Wärmebehandlung der Mutterlauge unter Freisetzung von CO₂ und Rückführung in Schritt 2:

   KHCO3 → 0,5 K₂CO₃ + 0,5 CO₂
Die "over all"-Gleichung der Methionin-Synthese nach dem obigen Prozess sieht wie folgt aus:

   MMP + HCN + H₂O → Methionin

Die Recyclierung von K₂CO₃ und NH₃ gemäß den oben erwähnten Teilschritten stellt kein Problem dar.

Betrachtet man jedoch die Schritte 2 und 3 so ist erkennbar, dass aus diesen stöchiometrisch gesehen pro Mol NH₃ 1,5 Mole CO₂ freigesetzt werden. Gemäss EP 0 780 370 A können diese flüchtigen Komponenten in die Stufe der Hydantoinbildung (Schritt 1) zurückgeführt werden.

Gemäß der Stöchiometrie der Reaktion verlassen jedoch 0,5 Mole CO₂ diese Stufe ohne Umsetzung ungenutzt als Abgas.

Eine einfache Rückführung ist nicht möglich, da die chemische Analyse des Abgases ergibt, dass es, bezogen auf den CO₂ -Gehalt, Methylmercaptan (Mc) in Mengen von 1 - 10 Gew.-% enthält. Das Methylmercaptan-haltige Abgas kann nicht in der Methionin-Fällung (Schritt 4) genutzt werden, da das abfiltrierte Produkt stark geruchsbehaftet wäre. Darüber hinaus würde sich das Methylmercaptan wegen seines Säurecharakters als Kalium-Methylmercaptid in den Prozesslösungen anreichern, was zu Störungen der Methioninaufarbeitung führen würde. Der Methylmercaptan-Gehalt des Abgases aus der Hydantoin-Synthese hat zwei Ursachen. Zum einen enthält der als Rohstoff eingesetzte Methylmercaptopropionaldehyd (MMP) prozessbedingt immer einen gewissen Methylmercaptan-Restgehalt (s. US 4,048,232 und US 3,878,057) zum anderen kann Methylmercaptan durch thermische Zersetzung von Methionin hauptsächlich in der durch Kaliumcarbonat unterstützten Hydantoinhydrolyse (Schritt 2) entstehen. Die thermische Zersetzung des Methionins wird auch in EP 839 804 beschrieben.

Aus den genannten Gründen wird das Abgas nach dem Stand der Technik verbrannt, wobei CO₂ und Methylmercaptan verloren gehen und gleichzeitig erhebliche Kosten für die Verbrennung anfallen.

Aus der US 4,319,044 ist bereits ein Verfahren zur Herstellung von 5-(2-Methylmercaptan)hydantoin bekannt, bei dem man den in dem hauptsächlich aus CO₂ bestehenden Abgas enthaltenen Cyanwasserstoff und das Methylmercaptan durch eine mehrstufige Wäsche zurückgewinnt. Gemäss diesem Verfahren wird das Abgas zunächst entweder mit einer Ammoniumcarbonat- oder einer verdünnten Hydantoinlösung in Kontakt gebracht, um HCN zu binden. Die mit HCN beladene Lösung wird in die Hydantoinsynthese zurückgeführt. In einer zweiten Waschstufe wird das noch Methylmercaptan- und Ammoniak enthaltende Abgas mit Wasser gewaschen, um NH₃ zu entfernen. In der dritten Waschstufe wird das Abgas mit Methylmercaptopropionaldehyd (MMP) gegengewaschen, wobei das Methylmercaptan als Hemithioacetal gebunden wird. Nachteilig an diesem Prozess ist, dass CO₂ als der mengenmäßig bedeutsamste Anteil des Abgasstromes ungenutzt entweicht. Weiterhin entsteht im genannten Verfahren ein zusätzlicher Abfallstrom durch den Ausstoß der dort beschriebenen Wasserwäsche, der die toxischen Stoffe Ammoniak und Methylmercaptan enthält, dessen Entsorgung mit hohen Kosten verbunden ist.

Das in dem US-Patent 4,319,044 beschriebene Verfahren besitzt zusätzlich den Nachteil, dass der Abgasstrom im Verlauf des Waschprozesses zuletzt mit MMP in Kontakt kommt.

Damit ist er zumindest entsprechend dem spezifischen Dampfdruck mit MMP gesättigt und nur eingeschränkt recyclierbar.

Aufgabe der Erfindung ist es, hauptsächlich aus CO₂ bestehende Abgasströme so von den in ihnen enthaltenen chemischen Verbindungen zu reinigen, dass das gereinigte CO₂ anschließend in den Produktionsprozess zurückgeführt werden kann.

Gegenstand der Erfindung ist ein Verfahren zur Herstellung von Methionin bei dem man 3-Methylmercaptopropionaldehyd (MMP), Cyanwasserstoff, Ammoniak und Kohlendioxid oder solche Komponenten, aus denen die vorgenannten Komponenten herstellbar sind zum 5-(2-Methylmercaptoethyl)-hydantoin umsetzt, dieses hydrolysiert und das Methionin unter Einleiten von CO₂ ausfällt, dadurch gekennzeichnet, dass man das aus dem Hydantoinsynthesereaktor austretende CO₂- und Methylmercaptan-haltige Abgas in der angegebenen Reihenfolge
a) mit Wasser oder mit Wasser, in dem Methylmercaptopropionaldehyd (MMP) maximal bis zur Löslichkeitsgrenze gelöst vorliegt,
b) anschließend mit MMP und
c) danach mit Wasser wäscht,
und die erhaltenen Waschflüssigkeiten in den Herstellprozess für Methionin oder seine Vorprodukte zurückführt,
und
den gereinigten CO₂-Gasstrom in einen eine Alkalisalzlösung des Methionins enthaltenden Reaktor einleitet, und Methionin aus diesem Salz ausfällt.

Das Verfahren ist besonders geeignet, wenn das Gas einen oder mehrere zusätzliche Anteile, ausgewählt aus der Gruppe Blausäure, Ammoniak und Wasser enthält.

Das aus dem Hydantoinsynthesereaktor austretende Abgas enthält im allgemeinen 60 bis 75 Gew.-% CO₂, 0,01 bis 0,1 Gew.-% Blausäure, 1 bis 10 Gew.-% Methylmercaptan, 0,5 bis 5 Gew.-% Ammoniak und 15 bis 25 Gew.-% Wasser.

Die Ammoniak enthaltende Waschflüssigkeit aus dem Waschschritt a) wird in den Hydantoinsynthesereaktor eingeleitet.

Die Methylmercaptan enthaltende Waschflüssigkeit aus dem Waschschritt b) wird in die MMP-Synthese eingeleitet, und die MMP enthaltende Waschflüssigkeit aus dem Waschschritt c)als Waschflüssigkeit für den Waschschritt a) verwendet.

In Waschschritt b) wählt man die Menge des eingesetzten MMP so, dass das Molverhältnis von MMP:Methylmercaptan (gasförmig) bei 1:1 bis 3:1, insbesondere 1:1 bis 2:1 liegt.

Die Wäsche wird bei einer Temperatur von 10 bis 60°C, insbesondere 10 bis 40°C durchgeführt.

Der Druck in der Waschvorrichtung liegt im allgemeinen um 1 bis 10 bar höher als der im Fällungsreaktor für Methionin. Geeignet als Waschvorrichtungen sind insbesondere mehrstufige Kolonnen mit Zwischensümpfen oder Kolonnen mit Ventil- oder Glockenböden.

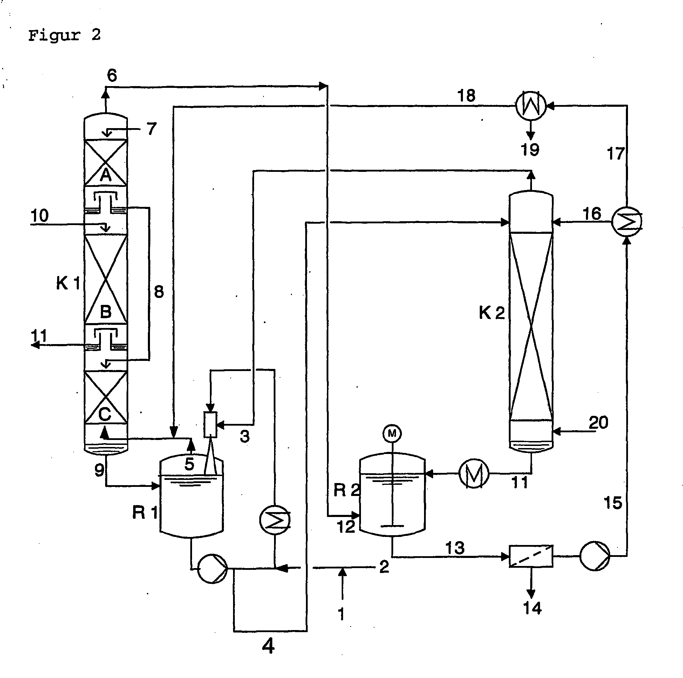

Die Wäsche erfolgt im Gegenstrom. Das gereinigte Gas hat einen CO₂-Gehalt von > 98 Gew.-%, insbesondere > 99 Gew.-% bis 99,8 Gew.-%. Zusätzlich kann auch das aus der Aufarbeitung der im Methioninprozess anfallenden Mutterlauge anfallende CO₂-haltige Gas (Schritt 6), aus dem das Wasser auskondensiert wurde, durch die Waschvorrichtung geleitet werden (Abbildung 2).

5-(2-Methylmercaptoethyl)-hydantoin wird gemäß EP 0 780 370 A hergestellt, indem eine Lösung von Cyanwasserstoff in 3-Methylmercaptopropionaldehyd und eine Lösung von Ammoniak und Kohlendioxid in Wasser bereitet wird, diese Lösungen schnell und innig miteinander vermischt und zur Umsetzung gebracht werden. Die Lösung von Cyanwasserstoff in 3-Methylmercaptopropionaldehyd wird zweckmäßigerweise so eingestellt, daß sie aus equimolaren Anteilen Cyanwasserstoff und 3-Methylmercaptopropionaldehyd besteht, oder aber überschüssige Anteile an Cyanwasserstoff enthält. Im allgemeinen ist es vorteilhaft, den Anteil Cyanwasserstoff in der Lösung nicht größer als 1.1 Mol je Mol 3-Methylmercaptopropionaldehyd zu wählen; vorzugsweise enthält die Lösung 1,005 bis 1,05 Mol Cyanwasserstoff je Mol 3-Methylmercaptopropionaldehyd.

Bei der Lösung von Ammoniak und Kohlendioxid in Wasser kann es sich um eine gesättigte oder verdünnte Lösung handeln; vorteilhaft ist der Gehalt an Ammoniak nicht unter etwa 5 Gew.-%. Das Molverhältnis Ammoniak zu Kohlendioxid beträgt zweckmäßigerweise etwa 1,2 bis 4,0 Mol, vorzugsweise 1,6 bis 1,8 Mol Ammoniak je Mol Kohlendioxid. Die Lösung des Cyanwasserstoffs in 3-Methylmercaptopropionaldehyd wird mit der Lösung des Ammoniaks und Kohlendioxids in Wasser so vermischt, dass in der Mischung zweckmäßigerweise ein Molverhältnis Ammoniak zu 3-Methylmercaptopropionaldehyd von etwa 1,2 bis 6 zu 1,0, vorzugsweise 2,0 bis 4,0 zu 1,0, insbesondere 2,5 bis 3,0 zu 1,0, vorliegt. Die Umsetzung wird bei Umgebungstemperatur oder darüber, zweckmäßigerweise bei Temperaturen über 60°C, vorteilhaft etwa zwischen 80°C und 140°C, ausgeführt. Vorzugsweise werden Temperaturen zwischen 80 und 130°C, insbesondere zwischen 90 und 120°C, gewählt. Wenngleich die Umsetzung bei beliebigem Druck ablaufen kann, ist es zweckmäßig, bei erhöhtem Druck zu arbeiten; vorteilhaft erweisen sich Drücke bis 20 bar, insbesondere Drücke, die 2 bis 3 bar über dem Gleichgewichtsdruck des Umsetzungsgemisches liegen. Die Umsetzungszeit richtet sich nach den Umsetzungsbedingungen, insbesondere nach der Temperatur und nach den Mengenverhältnissen.

Bei der bevorzugten Arbeitsweise ist es besonders vorteilhaft, die Lösung des Cyanwasserstoffs in 3-Methylmercaptopropionaldehyd und die Lösung des Ammoniaks und Kohlendioxids im Wasser in ein Umsetzungsgemisch dieser Substanzen, d. h. in ein zuvor bei der Umsetzung der Lösungen entstandenes Gemisch, in dem ganz oder teilweise die Umsetzung vom Hydantoin erfolgt ist, einzutragen und in diesem Gemisch die Umsetzung auszuführen.

Besonders vorteilhaft ist es, eine kontinuierliche Arbeitsweise zu wählen, hierzu das Umsetzungsgemisch im Kreislauf zu führen, in diesem Kreislauf an zwei benachbarten Stellen stetig die Lösungen von Cyanwasserstoff in 3-Methylmercaptopropionaldehyd und vom Ammoniak und Kohlendioxid in Wasser einzuspeisen und an einer anderen Stelle stetig aus dem Kreislauf einen entsprechenden Anteil des Umsetzungsgemisches abzuziehen.

Das Verfahren zur Herstellung von Methionin oder eines Alkalisalzes von Methionin durch Hydrolyse von 5-(2-Methylmercaptoethyl)-hydantoin in Gegenwart einer wässrigen Lösung enthaltend Alkali und Kohlendioxid, und ggf. weitere Umsetzung zum Methionin, wobei die Hydrolyse zumindest zu Beginn in Gegenwart von mind. 0,1 eq, insbesondere bis 7 eq, Ammoniak je Equivalent 5-(2-Methylmercaptoethyl)-hydantoin durchgeführt wird, ist ebenfalls aus der EP-Schrift bekannt.

Es hat sich gezeigt, dass es besonders vorteilhaft ist, wenn die Hydrolyse von Anfang an in Gegenwart von Alkali und Kohlendioxid, d. h., dass insbesondere eine Mischung von Alkaliverbindungen vorliegt, insbesondere Alkalihydrogencarbonat, Alkalicarbonat, Alkalihydroxid, wobei Alkali insbesondere für Kalium und Natrium steht, durchgeführt wird. Die Menge von Alkali und Kohlendioxid ist dabei zweckmäßig wenigstens die auf das Hydantoin bezogene stöchiometrische Menge. Diese kann nach oben deutlich überschritten werden. Ein Molverhältnis mit einem Überschuss von etwa 3 : 1 bezogen auf das Hydantoin ist besonders vorteilig; grundsätzlich ist davon auszugehen, dass ein noch größerer Überschuss noch günstiger ist. Für die Praxis jedoch sind Verhältnisse von etwa 1,5 : 1 - 2 : 1 besonders bevorzugt. Erfindungsgemäß wird dabei noch zusätzlich etwas Ammoniak zugegeben, das entsprechend ebenfalls teils auch in Form von Ammoniumverbindungen vorliegt. Besonders vorteilhaft ist es hierbei, wenn zu Beginn der Hydrolyse je Mol 5-(2-Methylmercaptoethyl)-hydantoin max. 7 Mol Ammoniak (incl. Ammoniumverbindungen) vorliegt. Hierdurch wird erreicht, dass die Hydrolyse praktisch ohne Nebenproduktbildung und in guten Ausbeuten abläuft und andererseits nicht oder nur wenig Alkalicarbonat ausfällt. Besonders vorteilhaft ist hierbei, wenn während der Hydrolyse Ammoniak und/oder Kohlendioxid, ggf. zusammen mit Wasser, aus dem Reaktionssystem ausgeschleust werden. Hierdurch lassen sich die Reaktionsbedingungen besonders günstig steuern, so dass weiterhin kein Alkalicarbonat ausfällt und die Reaktion vollständig verläuft.

Die Hydrolyseverfahren werden günstigerweise bei einer Temperatur von 120 bis 250°C und entsprechend einem Druck von 5 bis 30 bar durchgeführt. In diesem Bereich ergeben sich sehr gute Umsetzungen und geringe Nebenproduktbildung. Vorteilhaft ist auch, wenn die Alkalikomponente zumindest equimolar bezüglich des 5-(2-Methylmercaptoethyl)-hydantoins eingesetzt wird. Man erhält dann neben der vollständigen Hydrolyse auch praktisch quantitativ das entsprechende Alkalisalz des Methionins. Bevorzugt enthält die Hydrolyselösung zu Beginn auch bereits Methionin bzw. dessen Salz, auch dies hat einen günstigen, vermutlich autokatalytischen Effekt auf die Hydrolyse.

Bei dieser Verfahrensweise kann vorteilhaft im Laufe bzw. nach der Hydrolyse praktisch das gesamte Ammoniak und das gesamte Kohlendioxid aus der Hydrolyselösung abgezogen werden, so dass das Hydrolysat im wesentlichen frei von Ammoniak und Kohlendioxid entnommen werden kann.

Auch hier ist es besonders vorteilhaft, das Verfahren kontinuierlich durchzuführen. Ganz besonders vorteilhaft ist hierbei, dass die bis jetzt beschriebenen Verfahren zusammengeschaltet werden können, insbesondere als gesamtkontinuierlicher Prozeß, wobei Kohlendioxid und Ammoniak recycliert werden können.

Methionin wird aus Alkalimethioninat in wässriger Lösung durch Einleiten von Kohlendioxid freigesetzt, wobei man die Freisetzung bevorzugt in einem Rührzellenreaktor mit intensiver Durchmischung oder in einem Rührreaktor mit quasi idealer Durchmischung durchführt.

Bei der Freisetzung des Methionins aus der wässrigen Lösung mittels Kohlendioxid ist es besonders vorteilhaft, wenn das Kohlendioxid über eine Düsenvorrichtung im Bereich des Bodens in die wässrige Lösung eingeleitet wird. Dies begünstigt wiederum die Freisetzung des Methionins. Außerdem führt man die Freisetzung vorteilhaft bei einem Druck von 1 bis 30 bar durch, vorzugsweise auch bei einer Temperatur von 0 bis 100°C.

Ganz besonders vorteilhaft wird eine wässrige Lösung eingesetzt, die im wesentlichen frei von Ammoniak ist.

Auch die letzte Verfahrensweise wird besonders günstig kontinuierlich durchgeführt.

Das erfindungsgemäße Vorgehen macht es möglich, das Methioninverfahren ohne CO₂-Verlust zu betreiben. Weitere unerwünschte Abgas- bzw. Abwasserströme fallen nicht an.

### Beispiele

### Beispiel 1

Wie in EP 0780370 beschrieben wird die Hydantoinhydrolyse in (K 2) bevorzugt bei einem Druck von 7 - 9 bar und die Freisetzung des Methionins aus seinem Kaliumsalz mit CO₂ in (R 2) bei einem Druck von 2 bis 5 bar durchgeführt. Daher ist es besonders vorteilhaft die Anlagenteile Hydantoinhydrolyse (K 2), Hydantoinreaktor (R 1) und das CO₂-Waschsystem gemeinsam auf einem höheren Druck als den Reaktor (R 2) zu halten, so dass das gereinigte CO₂ (6) ohne weitere Förderorgane in den Fällreaktor (R 2) gelangt. Weiterhin vorteilhaft ist es, die 3-stufige CO₂-Wäsche wie oben dargestellt in einer Kolonne mit zwei Zwischensümpfen zu vereinigen, um den apparativen Aufwand zu minimieren.

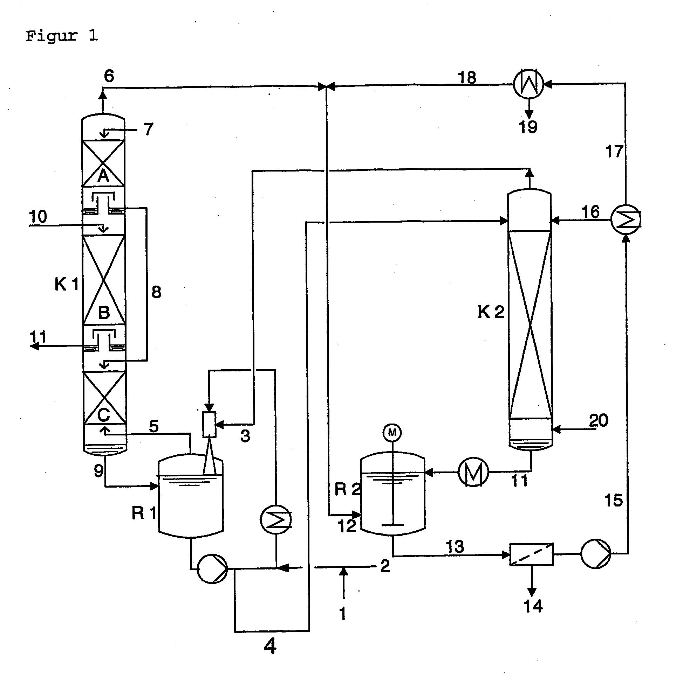

Anhand der Abbildung 1 und das eingangs aufgeführten Verfahrensschritten wird die Erfindung genauer erläutert:

Dem kontinuierlich betriebenen Hydantoinreaktor (R 1) werden HCN (1) und MMP (2) zugeführt (Schritt 1). Über 3 gelangen die Reaktionspartner NH₃ und CO₂ aus der Hydrolyse- und Strippkolonne (K 2) hinzu (Schritt 2 +3). Überschüssiges, Mc- und NH₃-haltiges CO₂ verlässt den Hydantoinreaktor (R1) über 5 und wird im unteren Teil der Waschkolonne (K 1, C) mit wässriger Lösung 8 NH₃-frei gewaschen. Anschließend tritt das Gas von unten in die Waschzone (K 1, B) ein und wird im Gegenstrom mit MMP (10) Mc-frei gewaschen. Zuletzt wird das Gas mit Wasser (7) feingereinigt und verlässt die Kolonne (K 1) über 6. Das mit Mc beladenen MMP (Hemithioacetal) verlässt die Kolonne (K 1) über 11 und wird der MMP-Synthese zugeführt.

Das gereinigte CO₂ wird mit dem aus der Aufarbeitung zurückgewonnen CO₂ (18) vereinigt und dient zur Fällung des Methionins aus der Hydrolyselösung (11) im Reaktor (R 2)(Schritt 4). Die Suspension (13) wird filtriert, der Filterkuchen (14) der weiteren Aufarbeitung zugeführt (Schritt 5). Die Filtrate (15) werden eingedampft (Schritt 6). Das Konzentrat (16) dient als K₂CO₃-Quelle für die Hydantoinhydrolyse in der Kolonne (K 2). Die Energiezufuhr und der Strippeffekt wird durch die Dampfzufuhr 20 bewirkt (Schritt 2 + 3).

Aus den Brüden der Eindampfung wird Wasser (19) auskondensiert und das danach verbleibende CO₂ (18) wird mit dem CO₂ aus der Waschkolonne (K 1) vereinigt dem Reaktor R 2 zugeführt (Schritt 4).

Besonders vorteilhaft lässt sich die Reaktivabsorption von Mc an MMP durchführen, wenn der Kolonnenteil (K 1, B) aus Ventil- oder Glockenböden oder einer vergleichbaren Konstruktion besteht. Diese technische Ausführung hat den Vorteil, dass sie ein minimales MMP zu Mc-Gas-Verhältnis ermöglicht. Dadurch wird der Aufwand zur Förderung der zum Waschen eingesetzten MMP-Menge minimiert. Im vorliegenden Fall gelingt es vorteilhafterweise den Mc-Anteil im CO₂-Gas mit einer minimalen, d.h. fast stöchiometrischen Menge an MMP quantitativ auszuwaschen.

Zusätzlich nicht genutzte Abgas-/Abwasserströme enthält dieses Verfahren nicht.

Es ist auch möglich, den bei der Aufarbeitung der Mutterlauge entstandenen CO₂-haltigen Abgasstrom ganz oder teilweise (Schritt 6) ebenfalls durch den Wäscher zu leiten (Abbildung 2).

Dies ist vorteilhaft, um das darin ebenfalls enthaltende Methylmercaptan zurückzugewinnen. Typischerweise enthält dieser Strom 97 bis 99 Gew.-% CO₂, 0,1 bis 1 Gew.-% Wasser und 0,1 bis 1 Gew.-% Methylmercaptan.

Dadurch verschiebt sich der CO₂-Gehalt im zu waschenden gesamten Abgasstrom im Vergleich zu dem Gehalt im Abgas aus dem Hydantoinsynthesereaktor zu höheren Werten. Die Verringerung der Konzentration der übrigen Bestandteile hat auf die Wirksamkeit der Wäsche keinen Einfluss.

### Beispiel 2

Eine wie in Abbildung 1 dargestellte Anlage wird mit einer Produktionsleistung von 100 kg/h Methionin betrieben.

Am Hydantoinreaktor R1 fällt dann bei 80°C eine Abgasmenge von 22,1 kg/h mit der Zusammensetzung 68 Gew.-% CO₂, 27 Gew.-% Wasser, 3,6 Gew.-% Methylmercaptan, 0,9 Gew.-% NH3 und 0,5 Gew.-% sonstige Komponenten an.

Dieses Rohgas wird in der dreistufigen Kolonne K1,A,B,C im Gegenstrom behandelt, wobei der Betriebsdruck am Kopf der Kolonne bei 6 barü liegt.

In Zone C der Kolonne K1 wäscht man den Gasstrom mit 15 kg/h Wasser bei 30°C, das aus dem Ablauf der Zone A bezogen wird.

Beim Eintritt in die Waschzone B der Kolonne K1 hat der Gasstrom die Zusammensetzung: 91 Gew.-% CO2, 4,8 Gew.-% Methylmercaptan, 3,6 Gew.-% Wasser und 0,4 Gew.-% sonstige Komponenten.

In der Waschzone B wird bei 20°C mit 2 kg/h Methylmercaptopropionaldehyd im Gegenstrom gewaschen. Danach hat der Gasstrom die Zusammensetzung 99,8 Gew.-% CO2, 0,01 Gew.-% MMP und < 0,2 Gew.-% sonstige Komponenten. Methylmercaptan ist gaschromatographisch nicht mehr nachweisbar.

Zur Feinreinigung wird das Gas noch durch die Zone A geführt und mit 15 kg/h Wasser von mitgeschlepptem MMP befreit.

Das so gereinigte Gas hat einen Gehalt von > 99,8 G% CO2.

## Patentansprüche

1. Verfahren zur Herstellung von Methionin bei dem man 3-Methylmercaptopropionaldehyd (MMP), Cyanwasserstoff, Ammoniak und Kohlendioxid oder solche Komponenten, aus denen die vorgenannten Komponenten herstellbar sind zum 5-(2-Methylmercaptoethyl)-hydantoin umsetzt, dieses hydrolysiert und das Methionin unter Einleiten von CO₂ ausfällt,
**dadurch gekennzeichnet, dass** man das aus dem Hydantoinsynthesereaktor austretende CO₂- und Methylmercaptan-haltige Abgas in der angegebenen Reihenfolge
a) mit Wasser oder mit Wasser, in dem Methylmercaptopropionaldehyd (MMP) maximal bis zur Löslichkeitsgrenze gelöst vorliegt,
b) anschließend mit MMP und
c) danach mit Wasser wäscht,
und die erhaltenen Waschflüssigkeiten in den Herstellprozess für Methionin oder seine Vorprodukte zurückführt,
und
den gereinigten CO₂-Gasstrom in einen eine Alkalisalzlösung des Methionins enthaltenden Reaktor einleitet, und Methionin aus diesem Salz ausfällt.

2. Verfahren gemäß Anspruch 1, bei dem man die Waschflüssigkeit aus dem ersten Waschschritt a) in den Hydantoinsynthesereaktor leitet.

3. Verfahren gemäss Anspruch 1, bei dem man die Waschflüssigkeit aus dem zweiten Waschschritt b) in die MMP-Synthese einleitet.

4. Verfahren gemäss Anspruch 1, bei dem man die Waschflüssigkeit aus dem dritten Waschschritt c)als Waschflüssigkeit für den Waschschritt a) verwendet.

5. Verfahren gemäss einem oder mehreren der Ansprüche 1 bis 4, bei dem man eine mehrstufige Kolonne mit Zwischensümpfen einsetzt und im Gegenstrom wäscht.

6. Verfahren nach Anspruch 5, bei dem man eine Kolonne mit Ventil- oder Glockenböden einsetzt.

7. Verfahren gemäss Anspruch 1, bei dem man in Waschschritt b) das Abgas mit MMP wäscht, wobei das Molverhältnis MMP:Methylmercaptan (gasförmig) bei 1:1 bis 2:1 liegt.

8. Verfahren gemäss einem oder mehreren der Ansprüche 1 bis 7, bei dem man das CO₂-haltige Abgas bei 10 bis 60 °C wäscht.

9. Verfahren gemäss einem oder mehreren der Ansprüche 1 bis 8, bei dem man das CO₂-haltige Abgas bei einem Druck wäscht, der 1 bis 10 bar höher ist als der im Fällungsreaktor für Methionin eingestellte.

10. Verfahren gemäß Anspruch 1, bei dem man das bei der Aufarbeitung der bei der Ausfällung des Methionins mit CO₂ anfallenden Mutterlauge entstehende CO₂-haltige Abgas ganz oder teilweise durch die Waschvorrichtung leitet.

## Claims

1. Process for preparing methionine in which 3-methylmercaptopropionaldehyde (MMP), hydrogen cyanide, ammonia and carbon dioxide or those components from which the aforementioned components can be prepared are converted to 5-(2-methylmercaptoethyl)hydantoin, this is hydrolysed and the methionine is precipitated with introduction of CO₂,
**characterized in that** the CO₂- and methyl mercaptan-containing offgas exiting from the hydantoin synthesis reactor is scrubbed in the specified sequence
a) with water or with water in which methylmercaptopropionaldehyde (MMP) is present dissolved up to a maximum of the solubility limit,
b) subsequently with MMP and
c) then with water,
and the resulting scrubbing liquids are recycled into the preparation process for methionine or its precursors,
and the purified CO₂ gas stream is introduced into an alkali metal salt solution of the methionine-containing reactor, and methionine is precipitated from this salt.

2. Process according to Claim 1, in which the scrubbing liquid from the first scrubbing step a) is passed into the hydantoin synthesis reactor.

3. Process according to Claim 1, in which the scrubbing liquid from the second scrubbing step b) is passed into the MMP synthesis.

4. Process according to Claim 1, in which the scrubbing liquid from the third scrubbing step c) is used as scrubbing liquid for the scrubbing step a).

5. Process according to one or more of Claims 1 to 4, in which a multistage column with intermediate column bottoms is used and scrubbing is effected in countercurrent.

6. Process according to Claim 5, in which a column having valve or bubble-cap trays is used.

7. Process according to Claim 1, in which the offgas is scrubbed in scrubbing step b) with MMP, the molar MMP:methylmercaptan (gaseous) ratio being 1:1 to 2:1.

8. Process according to one or more of Claims 1 to 7, in which the CO₂-containing offgas is scrubbed at 10 to 60°C.

9. Process according to one or more of Claims 1 to 8, in which the CO₂-containing offgas is scrubbed at a pressure which is 1 to 10 bar higher than that established in the precipitation reactor for methionine.

10. Process according to Claim 1, in which the CO₂-containing offgas formed in the workup of the mother liquor obtained in the precipitation of methionine with CO₂ is passed fully or partly through the scrubbing apparatus.

## Revendications

1. Procédé pour la production de méthionine, dans lequel on fait réagir du 3-méthylmercaptopropionaldéhyde (MMP), de l'acide cyanhydrique, de l'ammoniac et du dioxyde de carbone ou des composants à partir desquels peuvent être préparés les composants précités, pour obtenir de la 5- (2-méthylmercaptoéthyl) - hydantoïne, on hydrolyse cette dernière et on fait précipiter la méthionine en faisant passer du CO₂, **caractérisé en ce qu'**on lave le gaz rejeté contenant du méthylmercaptan et du CO₂ sortant du réacteur de synthèse d'hydantoïne, dans l'ordre indiqué
a) avec de l'eau ou avec de l'eau dans laquelle du méthylmercaptopropionaldéhyde (MMP) se trouve dissous jusqu'à la limite de solubilité,
b) ensuite avec MMP et
c) après avec de l'eau,
et on renvoie dans le processus de production de méthionine ou de ses précurseurs les liquides de lavage obtenus,
et
on fait passer le courant de gaz CO₂ épuré dans un réacteur contenant une solution de sel alcalin de la méthionine, et on fait précipiter la méthionine à partir de ce sel.

2. Procédé selon la revendication 1, dans lequel on fait passer dans le réacteur de synthèse d'hydantoïne le liquide de lavage provenant de la première étape de lavage a).

3. Procédé selon la revendication 1, dans lequel on envoie dans la synthèse de MMP le liquide de lavage provenant de la deuxième étape de lavage b).

4. Procédé selon la revendication 1, dans lequel on utilise le liquide de lavage provenant de la troisième étape de lavage c) en tant que liquide de lavage pour l'étape de lavage a).

5. Procédé selon une ou plusieurs des revendications 1 à 4, dans lequel on utilise une colonne à plusieurs étages à fonds intermédiaires et on lave à contre-courant.

6. Procédé selon la revendication 5, dans lequel on utilise une colonne à plateaux à cloches ou clapets.

7. Procédé selon la revendication 1, dans lequel dans l'étape de lavage b) on lave avec du MMP le gaz rejeté, le rapport molaire MMP:méthylmercaptan (gazeux) valant de 1:1 à 2:1.

8. Procédé selon une ou plusieurs des revendications 1 à 7, dans lequel on lave à une température de 10 à 60 °C le gaz rejeté contenant du CO₂.

9. Procédé selon une ou plusieurs des revendications 1 à 8, dans lequel on lave le gaz rejeté contenant du CO₂, sous une pression qui est de 1 à 10 bars supérieure à celle ajustée dans le réacteur de précipitation pour la méthionine.

10. Procédé selon la revendication 1, dans lequel on fait passer dans le dispositif de lavage en partie ou en totalité le gaz rejeté contenant du CO₂, formé lors du traitement final de la liqueur-mère produite dans la précipitation de la méthionine avec CO₂.
